# EUROPEAN PATENT APPLICATION

(11) **EP 4 684 732 A1**
(43) Date of publication of application: **28.01.2026**
(21) Application number: 24190320.2
(22) Date of filing: 23.07.2024
(51) Int. Cl.: A61B 6/00

(54) **METHOD AND PROCESSING APPARATUS FOR DETERMINING MEDICAL IMAGE ACQUISITION PARAMETERS USING ANATOMICAL INFORMATION**

(71) Applicant: Koninklijke Philips N.V., 5656 AG Eindhoven (NL)
(72) Inventor: BYSTROV, Daniel, Eindhoven (NL); VON BERG, Jens, Eindhoven (NL); KROENKE-HILLE, Sven, 5656AG Eindhoven (NL); YOUNG, Stewart Matthew, Eindhoven (NL); DESHPANDE, Hrishikesh Narayanrao, Eindhoven (NL); BERGTHOLDT, Martin, Eindhoven (NL); MENSER, Bernd, Eindhoven (NL); SENEGAS, Julien Thomas, Eindhoven (NL)
(74) Representative: Philips Intellectual Property & Standards

(57) **Abstract**

The invention concerns a method determining medical image acquisition parameter, comprising the steps of performing a first medical image acquisition from a first view of a subject using first acquisition parameter; receiving at least one first image from the first medical image acquisition; determine at least one anatomical structure in the first image; selecting a second medical image acquisition from a second view of the subject using second acquisition parameter; adjusting the second acquisition parameter for the second medical image acquisition in the second view of the subject based on the anatomical structure in the first image. The invention further concerns a processing apparatus configured to perform the method steps and a medical imaging system comprising the processing apparatus.

## Description

### FIELD OF THE INVENTION

The invention relates to the field of medical imaging, in particular to the field of medical image acquisition.

More specifically, the invention relates to the field of determining medical image acquisition parameters using anatomical information from a subject.

### BACKGROUND OF THE INVENTION

When examining a subject, such as a patient, for the inspection of a certain anatomy with a medical image system multiple views of the same anatomy may be necessary and are acquired using the medical imaging system. The subject is placed for the examination in different positions for being inspected in different views. The different position or views of the subject require an adaption of parameters used for the medical imaging system. These parameters are estimated from the outer physiognomy of the subject for example by the operator of the medical imaging system itself.

### SUMMARY OF THE INVENTION

Therefore, there exist a need for optimizing the adjustment of medical image acquisition parameters when performing an image acquisition in different views.

An object of the invention is to provide an improved adjustment of medical image parameters, by simultaneously reducing a dose of the subject undergoing medical image acquisition.

The object of the present invention is solved with the subject matter of the independent claims, wherein further embodiments are incorporated in the dependent claims.

According to a first aspect of the invention a method for determining medical image acquisition parameter is described. The method comprising the steps of performing a first medical image acquisition from a first view of a subject using first acquisition parameter; receiving at least one first image from the first medical image acquisition; determine at least one anatomical structure in the first image; selecting a second medical image acquisition from a second view of the subject using second acquisition parameter; adjusting the second acquisition parameter for the second medical image acquisition in the second view of the subject based on the anatomical structure in the first image.

In the context of the present invention, the term "acquisition parameter" shall be understood to describe a value of a medical image device and/or system, which needs to be set for performing a sufficient image acquisition.

In the context of the present invention, the term "view" shall be understood to describe a position of the subject arranged for medical image acquisition. This position or pose may depend on the examination which should be carried out and may be determined prior to the examination by a physician or medical staff, for example it may be determined with the prescription of the examination.

In the context of the present invention, the term "anatomical structure" shall be understood to describe, an inner structure of the subject, such as an inner anatomical part of the subject. For example, the anatomical structure may be a lung, a heart, or bones inside the subject, i.e., the patient. The anatomical structure may also be only a part of an inner structure, which means not the whole lung need to be investigated, such as the lower lung border may be sufficient. The relevant anatomical structure, or so-called target anatomy, may depend on the part of the subject to be examined, which may be predefined by the physician per medical prescription.

In other words, a subject, such as for example a patient, may be positioned for an image acquisition in a first view. Generally speaking, anatomical information may be extracted and may be used in a subsequent view acquisition. For a proper examination of the target anatomy of the subject, wherein the target anatomy may be the whole subject or only parts of the subject, such as head, chest, leg, knee, lung, heart, or the like, a new medical image may need to be taken. For this new medical image, the subject may be positioned in a different view than the first view. For example, this new different view, may be perpendicular to the first view. The number of views may be not limited, it may depend on the subject itself and the target anatomy which should be examined. Hence, more than two different views, for example, first view, second view, third view, fourth view, may be performed. It should be noted that the first view is different to the second view, wherein at least one first acquisition parameter may be different to the second acquisition parameter. This means that different acquisition parameters may be adjusted for different views. Hence, it should be noted that the acquisition parameter is not limited to one parameter. On the other hand, it may be possible that for the first and second view a value of one and the same acquisition parameter may be different and may be adjusted. It may also be possible that a value of the first acquisition parameter and a value of the second acquisition parameter may be the same or may only be slightly different. The term acquisition parameter may refer to different parameters which may be set before an acquisition could be performed. Differently speaking, it may also be possible for determining and adjusting a plurality of medical image acquisition parameters. Hence, more than one first acquisition parameter may be set and wherein all first set acquisition parameters may be different than all set second acquisition parameters. The second acquisition parameter are adapted by taking into account the anatomical structure from the first image. This allows a precise adaption of the acquisition parameters for each following view (pose) of the subject. Accordingly, it may be possible to reduce retakes of acquisition images. Further, a dose of ionizing radiation may be reduced as the following acquisitions can be adapted to the anatomy of the subject.

The described invention may serve for optimizing acquisition parameters during a single examination consisting of multiple different views without reproducing any previous examinations, images, and/or photographic conditions.

It should be noted that any feature, function, and/or element described in the following with reference to the processing apparatus equally applies to the method, and vice versa. Accordingly, any feature, function, step, and/or element described in the following with reference to one aspect of the present disclosure equally applies to any other aspect of the present disclosure.

According to an exemplary embodiment of the invention, the medical image acquisition may be an X-ray acquisition, wherein the medical image acquisition parameter may be an X-ray acquisition parameter.

The method may be applicable to all medical imaging processes from which images are generated comprising information about an inner structure of a subject. However, the X-ray imaging may be the imaging process of choice, as from a first X-ray image obtained with an X-ray image system using first X-ray acquisition parameters inner anatomical structures can be determined. In particular, the first acquisition parameters may be first X-ray acquisition parameters, the second acquisition parameters may be second X-ray acquisition parameters, the first image may be a first X-ray image, the second image may be a second X-ray image.

According to an exemplary embodiment of the invention, the acquisition parameter may be at least one of a radiation dose, a collimation parameter, a collimation area, a source image distance, an exposure time, a tube voltage, or a tube current.

As described in another embodiment, the acquisition parameter may be at least one of the above-mentioned setting parameters of a medical imaging system. In particular, the parameter may be an image source parameter. For example, the collimation parameter may be a collimation area, or a collimation region defined for being collimated. The area may be defined by collimation blades which allow radiation to pass through and onto the subject under acquisition. A collimation parameter may be understood as a parameter used to adjust a collimation of the medical image device, for example a parameter to adjust a collimator, and/or one or more collimator blades; or it may be an electrical parameter. The tube current may be the ms tube current.

According to an exemplary embodiment of the invention, a first view and/or a second view may be a predetermined view of the subject from a certain radiation source direction towards the subject depending on medical examination to be performed.

For example, a view may be at least one of the following views: an anterior-posterior view of the subject, a posterior-anterior view of the subject, a lateral view of the subject, an oblique view of the subject, a mortise view of the subject, or a sunrise view of the subject. Anterior describes a view from the front of the subject, while posterior describes a view from a back of the subject. Superior describes the top of a part of the subject, while inferior describes the bottom of the subject. The posterior-anterior view may be described as, during the examination process of taking an X-ray image, for example, the radiation passes from posterior of the subject to anterior of the subject. An oblique chest view is a type of imaging that comprises obtaining a medical image of the chest from an angle, wherein the subject may be positioned at a slight angle to the image system.

According to an exemplary embodiment of the invention, one or more previously acquired image is processed for determining one or more anatomical structure and afterwards one or more acquisition parameter is determined for a subsequent medical image acquisition.

In other words, any amount of previously acquired images may be used. On these images one or also a plurality of anatomical structures can be detected, and/or identified from which in turn one or also a plurality of acquisition parameter(s) may be determined for a further image acquisition. The previously acquired image may be the first image, the second image, or both or any other image if more than one image have been acquired.

For example, more than one image is processed for determining one and the same anatomical structure from this plurality of images. On the other hand, from the plurality of images different, hence more than one, anatomical structures may be determined. It may also be possible that from one image a plurality of anatomical structures may be determined.

According to an exemplary embodiment of the invention, the method may further comprise the step of selecting a third medical image acquisition from a third view of the subject using third acquisition parameter. The method may further comprise an additional step of adjusting the third acquisition parameter for the third medical image acquisition from the third view of the subject based on imaged anatomical structures in the first image and the second image.

The method may comprise more steps for selecting a plurality of medical acquisition and may not be limited to one, two or three. In particular, the amount of medical image acquisition may depend on the necessary views determined by a physician or the medical staff performing the method. The step of adjusting the third acquisition parameter for the third medical image acquisition may be described as adjusting from the third view of the subject based on mapped anatomical structures in the first image and the second image. The mapping may be understood that a physical object may be mapped to the X-ray detector.

According to an exemplary embodiment of the invention, the method may further comprise the step of determine a further anatomical structure in the second image, wherein the step of adjusting the third acquisition parameter is additionally or alternatively based on the adapted second acquisition parameter.

For instance, if it is necessary to perform an image acquisition comprising three different views of the subject, i.e., of the patient or parts from the patient, the method may determine an anatomical structure in the first image in a first view. From the first determined anatomical structure the acquisition parameter for the subsequent view may be determined. These acquisition parameters are used for setting the medical image device for a second image in a second view. From this second image one or more further (second) anatomical structure(s) may be determined and from this/these anatomical structure the third acquisition parameters may be derived by a computing unit, e.g. a neural network. It may also be possible that the third acquisition parameters may be derived from the anatomical structures of the first image or from both determined anatomical structures of both images, the first and the second image.

According to an exemplary embodiment of the invention, the step of determining the anatomical structure may comprise applying an advanced artificial intelligence method for determining the anatomical structure in the preceding image.

The preceding image may be the first image, the second image or both images. It may also be possible that more than two images are used.

For example, a machine leaning algorithm, or a neural network, or a combination thereof may be applied for determining the anatomical structure in the respective picture. Depending on the relevant anatomical structure different approaches may be applied. The determination of the anatomical structure may be performed automatically by a processor unit.

According to an exemplary embodiment of the invention, the artificial intelligence method may be a trained neural network, which may be trained with a plurality of medical images from different views from a subject for determining anatomical structures from the medical image.

The respective artificial intelligence method may be trained with previously taken images, such as X-ray images of one and the same anatomical structure taken in different views. A plurality of training data may be provided, wherein the training data may comprise a plurality of images from different patients of one and the same anatomical structure in the same view and/or in different views.

According to an exemplary embodiment of the invention, the method may further comprise the step of receiving a subject image from an optical image device, wherein the optical image device may be in particular a camera configured to receive the subject image from the subject. Further, the subject image may comprise image data about at least one of a contour, a surface, or geometric information of the subject.

The optical image device may provide more information, for example information about outer features of the subject. The optical image device may provide as an additional feature. For example, a height of the subject may help to estimate an appropriate position of the anatomical structure in the subject. Furthermore, the optical image device may help to compensate some geometric distortions by the different views/poses.

According to an exemplary embodiment of the invention, the optical image device may be a RGB camera, or a 3D depth camera. For example, for a posterior-anterior and lateral view an RGB-depth camera may help to compensate geometric distortions for example to determine lateral collimation borders for the image acquisition.

According to an exemplary embodiment of the invention, the method may further comprise the steps of performing a first medical image acquisition in the first view using the first image acquisition parameters, and/ or performing a second medical image acquisition in the second view using the second image acquisition parameters.

Differently speaking, the method may be extended to add steps for performing the medical image acquisition. The method may be adapted to be performed in a system configured for carrying out the image acquisition. The steps may depend on the number of images, hence the different view, which should be taken from the subject.

According to an exemplary embodiment of the invention, the step of adjusting the acquisition parameters such as for example X-ray dose or collimation, may be performed after detection of the anatomical structure, for example a size of the anatomical structure, by applying an advanced artificial intelligence method.

The advanced artificial intelligence method, such as machine learning, a neural network, may be the same advanced artificial intelligence method used for determining the anatomical structure. On the other hand, the artificial intelligence method may differ from the method used for determining the anatomical structure. The determined parameter may be used for the further image acquisition. A respective computing unit may be configured for performing the artificial intelligence method as described herein.

According to a second aspect of the invention, a medical imaging system is described. The system comprises an X-ray source and an X-ray detector for performing a first medical image acquisition from a first view of a subject using a first acquisition parameter and for performing a second medical image acquisition from a second view of the subject using a second acquisition parameter, The system further comprises a processing apparatus configured to receive at least one first image from the first medical image acquisition from the first view of a subject using first acquisition parameter, determine at least one anatomical structure in the first image, select the second medical image acquisition in the second view of the subject using the second acquisition parameter, adjust the second acquisition parameter for the second medical image acquisition from the second view of the subject based anatomical structure in the first image. The system, particularly the processor, may further be configured to determine the first acquisition parameter which is set for the first medical image acquisition. The first acquisition parameter may also be determined or set by the medical staff or the physician itself.

According to a third aspect of the invention, a data processing apparatus is described, comprising elements for carrying out the method according to any one of the embodiments as described herein.

The data processing apparatus may be an external apparatus, which is configured and able to control the medical imaging process. For example, the data processing apparatus may be in wireless communication with the medical imaging system. On the other hand, the data processing apparatus may be an integral part of a medical imaging device and/or system. The data processing apparatus may comprise a computing unit which uses, and/ or may be configured to use, and/or to perform, the artificial intelligence method, such as a neural network, as described in the exemplary embodiments herein.

According to a fourth aspect of the invention a computer program comprising instructions, which, when the program is executed by a computer, causes the computer to carry out the method as described herein with exemplary embodiments. In detail, the program may cause the computer to carry out the method for determining medical image acquisition parameter.

For example, the method and/or the computer program element may be implemented as part of the operating system of the device, such as the processing apparatus or the medical imaging system.

According to a fifth aspect of the invention, A computer-readable medium comprising instructions which, when executed by a computer, cause the computer to carry out the method of any one of the embodiments as described herein. In detail, the computer-readable medium may cause the computer to carry out the method for determining medical image acquisition parameter with any one of the embodiments herein.

It has to be noted that embodiments of the invention have been described with reference to different subject matters. In particular, some embodiments have been described with reference to device/apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the device/apparatus type claims and features of the method type claims is considered as to be disclosed with this application.

It should be noted that the term "comprising" does not exclude other elements or steps and "a" or "an" does not exclude a plurality. Also, elements described in association with different embodiments may be combined. It should also be noted that reference signs in the claims should not be construed as limiting the scope of the claims.

### BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates schematically a flow diagram describing steps of the method according to an exemplary embodiment of the invention.
Fig. 2 illustrates a schematically arrangement of a subject for a first view according to an exemplary embodiment of the invention.
Fig. 3 illustrates a schematically arrangement of the subject for a second view according to an exemplary embodiment of the invention.

### DETAILED DESCRIPTION OF EMBODIMENTS

The illustrations in the drawings are schematic. It is noted that in different figures similar or identical elements are provided with the same reference signs.

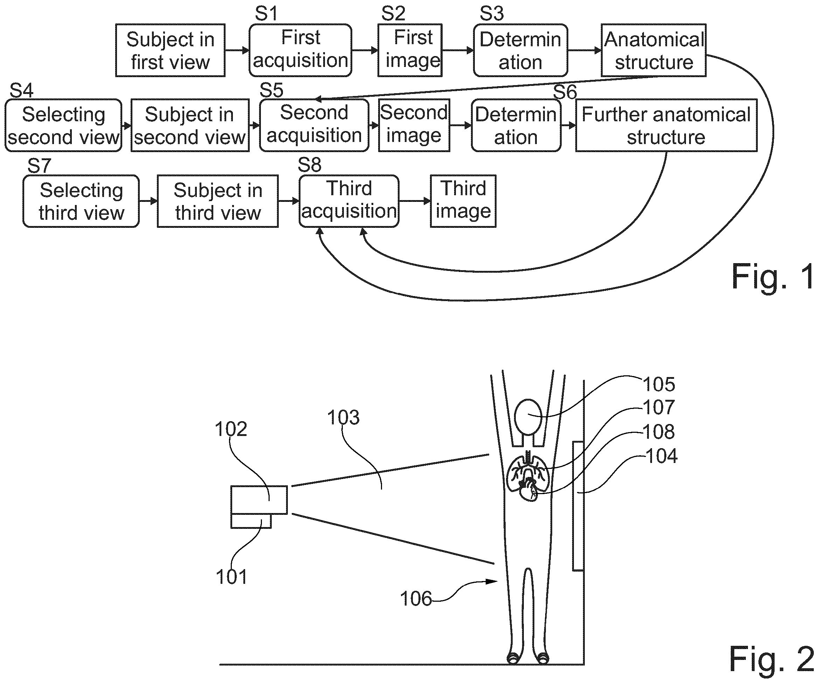

Fig 1 illustrates schematically a flow diagram describing steps of the method. The method for determining medical image acquisition parameter, comprises the steps of performing a first medical image acquisition S1 from a first view of a subject using first acquisition parameter, receiving at least one first image S2 from the first medical image acquisition S1, determine at least one anatomical structure S3 in the first image. As a result from step S3, which is the determination of the anatomical structure, the determined anatomical structure can be received by the processor. The data about the anatomical structure, such as the size, width, and/or height of the anatomical structure may be received and may be stored in an internal or external storage unit. The method further comprises the step of selecting a second medical image acquisition from a second view S4 of the subject using second acquisition parameter S5, adjusting the second acquisition parameter S5 for the second medical image acquisition in the second view of the subject based on the anatomical structure in the first image (which is indicated with the arrow in Figure 1). Furthermore, the method may proceed with the step S6 of determining a further (second) anatomical structure in the second image, wherein the result may be the size, width, height of the further anatomical structure. If a third image with a third view may be necessary, the method may continue with selecting of the third view S7 for the third image. The subject is therefore positioned in the third view. Then the third image acquisition S8 is performed, and a third image is received. The method may not be limited to the illustrated steps, further steps may be added, such as steps for performing a fourth image in a fourth view. On the other hand, the method may only comprise steps for a first image in a first view and a second image in a second view.

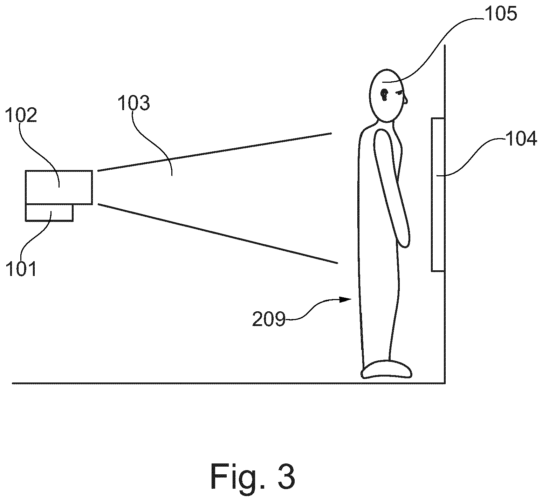

Fig. 2 illustrates a schematically arrangement of a subject for a first view. In figure 2 a processing apparatus 101 is illustrated, which apparatus 101 may perform the method as described with the exemplary embodiments herein. The processing apparatus can be attached to the medical imaging device(system). For example, in Figure 2 and also in Figure 3 the processing apparatus 101 is attached to the radiation source 102. The processing apparatus 101 may be an external or an internal part of the medical imaging device. As a medical imaging device an X-ray device or system is used. The subject, such as the patient 105, is positioned between the radiation source 102 and the detector 104. In Figure 1 the subject 105 is positioned in the lateral view 106, this means that the subject 105 is exposed by the radiation source 102 from the side. The medical imaging acquisition may be performed in the lateral view 106 for receiving a first image. From the first image the position an anatomical structure 107, and/or 108, such as the hearth 108 and/or the lung 107, may be determined. Further, the size of the anatomical structure 107, 108, or the height and/or width may be determined. The processing apparatus may further comprise an optical image device. The optical image device may be attached to the processing apparatus 101 or may be an integral part of the processing apparatus 101. For example, an external camera, as the optical image device may be attached to the radiation source 102 and/or the processing apparatus 101. The optical image device is arranged in such a manner that the subject 105 is in the field of view of the optical image device.

Fig. 3 illustrates a schematically arrangement of the subject for a second view. The second view may be the posterior anterior view 209, wherein the subject 105 is also positioned between the radiation source 102 and the detector 104. In the posterior anterior view, the subject 105 is irradiated by the radiation source 102 form the back side. This means that the radiation beam 103 passes through the subject 105 from the back side to the detector 104. When the lateral view 109 as illustrated in Figure 2 is the first view, the posterior anterior view 209 as illustrated in Figure 3 may be the second view. Hence, the method may perform the step of selecting a second medical image acquisition from a second view 209 of the subject 105 using second acquisition parameter and adjusting the second acquisition parameter for the second medical image acquisition in the second view of the subject based on the anatomical structure in the first image. As acquisition parameter to be adjusted, the radiation dose of the radiation source 102, the collimation of the radiation source 102, the source image distance, an exposure time, a tube voltage, and/or the tube current can be adjusted.

### LIST OF REFERENCE SIGNS:

- 101: processing apparatus
- 102: radiation source
- 103: radiation beam
- 104: detector
- 105: subject
- 106: lateral view
- 107: anatomical structure
- 108: further anatomical structure
- 209: posterior anterior view
- S1-S8: method steps

## Claims

1. Method for determining medical image acquisition parameter, comprising the steps of
performing a first medical image acquisition from a first view of a subject using first acquisition parameter;
receiving at least one first image from the first medical image acquisition;
determine at least one anatomical structure in the first image;
selecting a second medical image acquisition from a second view of the subject using second acquisition parameter;
adjusting the second acquisition parameter for the second medical image acquisition in the second view of the subject based on the anatomical structure in the first image.

2. The method according to claim 1,
wherein the medical image acquisition is an X-ray acquisition;
wherein the medical image acquisition parameter is an X-ray acquisition parameter.

3. The method according to claims 1 or 2,
wherein the acquisition parameter is at least one of a radiation dose, a collimation parameter, a collimation area, a source image distance, an exposure time, a tube voltage, or a tube current.

4. The method according to any of the preceding claims,
wherein one or more previously acquired image is processed for determining one or more anatomical structure and afterwards one or more acquisition parameter is determined for a subsequent medical image acquisition.

5. The method according to any of the preceding claims, further comprising the step of
selecting a third medical image acquisition from a third view of the subject using third acquisition parameter;
adjusting the third acquisition parameter for the third medical image acquisition from the third view of the subject based on imaged anatomical structures in the first image and the second image.

6. The method according to claim 5, further comprising the step of
determine a further anatomical structure in the second image;
adjust the second acquisition parameter based on the further anatomical structure;
wherein the step of adjusting the third acquisition parameter is additionally or alternatively based on the adapted second acquisition parameter.

7. The method according to any of the preceding claims,
wherein the step of determining the anatomical structure comprises applying an advanced artificial intelligence method for determining the anatomical structure in the preceding images.

8. The method according to claim 7,
wherein the artificial intelligence method is a trained neural network, which is trained with a plurality of medical images from different views from a subject for determining anatomical structures from the medical image.

9. The method according to any of the preceding claims, further comprising the step of
receiving a subject image from an optical image device, wherein the optical image device is in particular a camera configured to receive the subject image, wherein the subject image comprises image data about at least one of a contour, a surface, or geometric information of the subject.

10. The method according to claim 9,
wherein the optical image device is a RGB camera, or a 3D depth camera.

11. The method according to any of the preceding claims, further comprising the step of
performing a first medical image acquisition in the first view using the first image acquisition parameters,
performing a second medical image acquisition in the second view using the second image acquisition parameters.

12. The method according to any of the preceding claims 2 to 11,
wherein the step of adjusting the acquisition parameters is performed after detection of the anatomical structure by applying an advanced artificial intelligence method.

13. A medical imaging system, comprising
an X-ray source and an X-ray detector for performing a first medical image acquisition from a first view of a subject using a first acquisition parameter and for performing a second medical image acquisition from a second view of the subject using a second acquisition parameter,
a processing apparatus configured to
receiving at least one first image from the first medical image acquisition from the first view of a subject using first acquisition parameter;
determine at least one anatomical structure in the first image;
select the second medical image acquisition in the second view of the subject using the second acquisition parameter;
adjust the second acquisition parameter for the second medical image acquisition from the second view of the subject based anatomical structure in the first image.

14. A data processing apparatus comprising elements for carrying out the method according to any one of claims 1 to 12.

15. A computer program comprising instructions, which, when the program is executed by a computer, cause the computer to carry out the method according to any one of the claims 1 to 12.
